# EUROPÄISCHE PATENTSCHRIFT

(11) **EP 0 568 873 B1**
(45) Veröffentlichungstag und Bekanntmachung des Hinweises auf die Patenterteilung: **03.01.1996**
(21) Anmeldenummer: 93106596.5
(22) Anmeldetag: 23.04.1993
(51) Int. Cl.: C07C 255/24, C07C 211/11, C10L 1/22, C10M 133/54, C08F 8/30

(54) **Beta-Aminonitrile und N-Alkyl-1,3-propylendiamine sowie deren Verwendung als Kraft- und Schmierstoffadditive**
Beta-aminonitriles and N-alkyl-1,3-propylenediamines as well as their use as fuel and lubricant additives
Bêta-aminonitriles et N-alkyle-1,3-propylenediamines ainsi que leur utilisation comme additives de combustibles et de lubrifiants

(30) Priorität: 04.05.1992 DE 4214810
(43) Veröffentlichungstag der Anmeldung: 10.11.1993
(73) Patentinhaber: BASF Aktiengesellschaft, D-67063 Ludwigshafen (DE)
(72) Erfinder: Mohr, Juergen, Dr., W-6718 Gruenstadt (DE); Oppenlaender, Knut, Dr., W-6700 Ludwigshafen (DE); Franz, Lothar, Dr., W-6704 Mutterstadt (DE); Thomas, Juergen, Dr., W-6701 Fussgoenheim (DE)

(56) Entgegenhaltungen:
- DE-A- 2 245 918
- DE-A- 3 611 230
- FR-A- 1 462 117
- FR-A- 2 333 853
- US-A- 3 189 652
- US-A- 3 244 491
- US-A- 3 565 804
- US-A- 4 332 595

## Beschreibung

Die vorliegende Erfindung betrifft β-Aminonitrile, die am Aminstickstoff durch einen langkettigen Alkylrest substituiert sind, N-Alkyl-1,3-proylendiamine, die durch Hydrierung aus den β-Aminonitrilen erhalten werden, Verfahren zur Herstellung der β-Aminonitrile und der N-Alkyl-1,3-propylendiamine sowie Kraft- und Schmierstoffe, die diese Verbindungen als Additive enthalten.

Vergaser und Einlaßsystem von Ottomotoren, aber auch Einspritzsysteme für die Kraftstoffdosierung in Otto- und Dieselmotoren werden in zunehmendem Maße durch Verunreinigungen belastet, die durch Staubteilchen aus der Luft, unverbrannte Kohlenwasserstoffreste aus dem Brennraum und die in den Vergaser geleiteten Kurbelwellengehäuseentlüftungsgase verursacht werden.

Diese Rückstände verschieben das Luft-Kraftstoffverhältnis im Leerlauf und im unteren Teillastbereich, so daß das Gemisch fetter, die Verbrennung unvollständiger und wiederum die Anteile unverbrannter oder teilverbrannter Kohlenwasserstoffe im Abgas größer werden und der Benzinverbrauch steigt.

Es ist bekannt, daß zur Vermeidung dieser Nachteile Kraftstoffadditive zur Reinhaltung von Ventilen und Vergaser bzw. Einspritzsystemen verwendet werden (vgl. z.B.: M. Rossenbeck in Katalysatoren, Tenside, Mineralöladditive, Hrsg. J. Falbe, U. Hasserodt, S. 223 f., G. Thieme Verlag, Stuttgart 1978).

Je nach Wirkungsweise, aber auch nach dem bevorzugten Wirkort solcher Detergens-Additive unterscheidet man zwei Generationen.

Die erste Additiv-Generation konnte nur die Bildung von Ablagerungen im Ansaugsystem verhindern, nicht aber bereits vorhandene Ablagerungen wieder entfernen, wohingegen die Additive der zweiten Generation beides bewirken können ("keep-clean-" und "clean-up-Effekt") und zwar insbesondere auch aufgrund ihrer günstigen thermooxidativen Eigenschaften an Zonen höherer Temperaturen, nämlich an den Einlaßventilen.

Das molekulare Bauprinzip dieser als Detergenzien wirkenden Additive kann verallgemeinernd angegeben werden als Verknüpfung polarer Strukturen mit meist höhermolekularen, unpolaren oder oleophilen Resten.

Vertreter der zweiten Additiv-Generation sind oft Produkte auf der Basis von Polyisobutenen im unpolaren Molekülteil. Hier wiederum heben sich Additive vom Polyisobutylamin-Typ besonders hervor.

Detergenzien vom Typ der Polyisobutylamine erhält man, ausgehend von Polyisobutenen, im wesentlichen nach zwei Verfahren.

Das erste verläuft über eine Chlorierung des polymeren Grundkörpers und anschließendem nukleophilen Ersatz durch Amine oder Ammoniak. Aufgrund der Herstellung (Anlagerung von Cl₂, Ersatz eines Chloratoms durch das Amin, Abspaltung von HCl) weisen diese Produkte eine Doppelbindung auf. Der Nachteil dieses Verfahrens ist vor allem die Verwendung von Chlor und das Auftreten von Chlor- und Chlorid-haltigen Produkten, die heute keinesfalls mehr erwünscht sind und soweit irgend möglich gemieden werden (DE-OS 21 29 491, DE-OS 22 45 918, US 3 565 804, US 3 438 757).

Im zweiten Verfahren wird ein reaktives Polyisobuten zunächst in einer Oxosynthese hydroformyliert danach in Anwesenheit von Ammoniak aminierend hydriert (DE-OS 36 11 220).

Letztere Produkte zeigen meist eine ausgezeichnete Wirksamkeit in der Ventil- und Vergaserreinhaltung, verhalten sich jedoch allenfalls neutral in ihrer Wirkung auf einen Motorschmierstoff, insbesondere hinsichtlich ihrer Schlammdispergierung.

Die US 4 332 595 beschreibt Polyetheramine, die über eine Cyanoethylierung hergestellt werden und daher ebenfalls kein Chlor enthalten. Diese Polyetheramine zeigen jedoch ausschließlich eine Detergens-Wirkung und sind in Ölen meist schlecht löslich.

Ein Problem, das in diesem Zusammenhang auftritt, ist die Beeinflussung des Schmierstoffs durch Kraftstoffadditive, die in geringer Menge aber stetig über den Brennraum in den Schmiermittelkreislauf eines Motors gelangen.

Auf keinen Fall dürfen solche Zusätze, einmal in den Schmierstoff gelangt, dessen Eigenschaften und Funktion negativ beeinflussen. Insbesondere im Hinblick auf die Bildung und Dispergierung des Ölschlamms wird daher auch der Einfluß der Kraftstoffadditive berücksichtigt. Die meisten der bekannten Detergenzien verhalten sich allerdings ölschlammneutral.

Möchte man darüber hinaus einen positiven Effekt des Kraftstoffs bzw. der darin enthaltenen Wirkstoffe auf den Schmierstoff erzielen, dann ist die zusätzliche Additivierung des Kraftstoffs mit dispergierenden Substanzen sinnvoll.

Ideal in diesem Zusammenhang und von besonderem technischen Interesse wären solche Stoffe, die gleichzeitig die Eigenschaften sowohl von Detergenzien als auch von Dispergatoren in sich vereinigen.

Es bestand daher die Aufgabe, Stoffe zur Verfügung zu stellen, die halogenfrei sind und als Additive in Kraftstoffen außer ihrer positiven Wirkung im Einlaßsystem eines Ottomotors zusätzlich eine ölschlamm-dispergierende Wirkung aufweisen.

Es wurde nun gefunden, daß diese Aufgabe gelöst wird sowohl durch β-Aminonitrile der folgenden Formel I 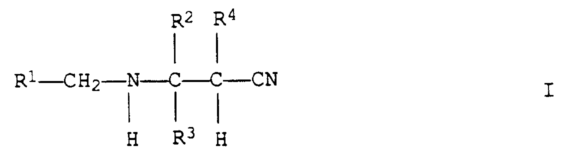
in der
- R¹: einen aliphatischen, Alkyl-Seitengruppen aufweisenden Kohlenwasserstoffrest mit einem Molekulargewicht (Zahlenmittel) von 250 bis 5 000 darstellt und
- R², R³ und R⁴: unabhängig voneinander Wasserstoff oder einen C₁-C₈-Alkylrest oder R² oder R⁴ einen Phenylrest bedeuten,
als auch durch
N-Alkyl-1,3-propylendiamine der folgenden Formel II 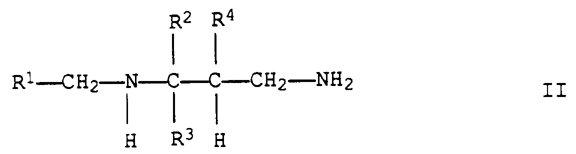
in der R¹ bis R⁴ die oben angegebene Bedeutung haben, und die durch Hydrierung der β-Aminonitrile der obigen Formel I an üblichen Hydrierkatalysatoren unter Druck bei Temperaturen von 50°C bis 300°C in Gegenwart von NH₃ erhalten werden.

Überraschenderweise stellt sich heraus, daß die erfindungsgemäßen Derivate von Polyisobutylaminen, im Gegensatz zu den ihnen zugrundeliegenden Aminen, neben ihrer ventilreinigenden Wirkung zusätzlich positiv auf das Schlammtragevermögen von schwach oder gar nicht additivierten Motorenölen einwirken. Diese Wirkung besteht gleichermaßen sowohl bei den cyanoethylierten als auch den durch deren Hydrierung erhaltenen aminopropylierten Vertretern.

Bei den Alkyl-Seitengruppen im Rest R¹ handelt es sich bevorzugt um verzweigte oder unverzweigte Alkyl-Reste mit 1 bis 30, insbesondere 1 bis 4 C-Atomen.

Besonders bevorzugt sind dabei Verbindungen, in denen jeweils R² und R³ Wasserstoff und R⁴ Wasserstoff oder Methyl bedeutet und/oder R¹ einen von Isobuten und 0 bis 30 Gew.-% n-Buten abgeleitetem Polybutyl- oder Polyisobutylrest darstellt.

Die erfindungsgemäßen Stoffe enthalten aufgrund ihrer Herstellung kein Halogen und weisen auch keine ungesättigten Anteile auf.

Dies wird dadurch erreicht, daß die Stoffe nach einem Verfahren hergestellt werden, das ohne Halogenierungszwischenschritte verläuft.

Gegenstand der Erfindung ist es somit auch ein Verfahren zur Herstellung von β-Aminonitrilen der obigen Formel I, das dadurch gekennzeichnet ist, daß man Amine der Formel III

R¹―CH₂―NH₂ III

mit Nitrilen der allgemeinen Formel IV 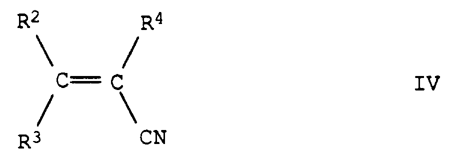
gegebenenfalls in Gegenwart eines Katalysators umsetzt, wobei R¹ bis R⁴ die oben angegebenen Bedeutungen haben.

Weiterhin ist Gegenstand der Erfindung ein Verfahren zur Herstellung von N-Alkyl-1,3-propylendiaminen der obigen Formel II, das dadurch gekennzeichnet ist, daß man β-Aminonitrile der Formel I an üblichen Hydrierkatalysatoren unter Druck bei Temperaturen von 50°C bis 300°C in Gegenwart von NH₃ hydriert.

Die als Ausgangsprodukte vorzugsweise eingesetzten Polyisobutylamine der obigen Formel III werden bevorzugt durch Hydroformylierung und anschließende Aminierung von Polyisobuten in an sich bekannter Weise erhalten.

Das hierzu verwendete Polyisobuten hat bevorzugt ein Molekulargewicht (Mn) zwischen 500 und 5 000, insbesondere zwischen 800 und 1 500. Es wird nach bekanntem Verfahren durch kationische Polymerisation von Isobuten erhalten, wobei nach Abbruch der Polymerkette im zuletzt eingebauten Monomeren eine Doppelbindung verbleibt, die zum Zwecke der weiteren Funktionalisierung genutzt werden kann (vgl. z.B.: DE-OS 27 02 604).

Die Cyanoethylierung des Polyisobutylamins erfolgt auf an sich bekannter Weise (s. z.B. Kirk-Othmer, Encyclopedia of Chemical Technology, 3. Ed., Vol. 7, p. 370-376, J. Wiley, 1979), z.B. durch Umsetzung des Amins mit Acrylnitril bei 50 bis 120°C.

Auch die katalytische Hydrierung der so erhaltenen Nitrile der Formel I zu den Aminen der Formel II wird auf an sich bekannter Weise durchgeführt, z.B. in einem Autoklaven bei Überdruck und Temperaturen von 50°C bis 300°C, vorzugsweise 80°C bis 150°C, in Gegenwart von Wasserstoff und NH₃ an üblichen Hydrierkatalysatoren, z.B. Raney-Cobalt oder Raney-Nickel.

Die erfindungsgemäßen β-Aminonitrile und/oder N-Alkyl-1,3-propylendiamine werden aufgrund ihrer Eigenschaften als Detergenzien und Dispergatoren in Kraftstoffen, insbesondere in Kraftstoffen von Ottomotoren, eingesetzt. Sie können jedoch auch in Schmierstoffen Verwendung finden.

Werden die β-Aminonitrile und/oder N-Alkyl-1,3-propylendiamine in Kraftstoffen eingesetzt, so gibt man sie bevorzugt in einer Menge von 10 bis 5 000 ppm, insbesondere 50 bis 1 000 ppm zu. In Schmierstoffen muß in der Regel höher additiviert werden, die Mengen können hier 0,1 bis 6 Gew.-%, insbesondere 0,5 bis 5 Gew.-% betragen.

Sollen in erster Linie die dispergierenden Eigenschaften der erfindungsgemäßen Stoffe genutzt werden, so kann man sie auch mit herkömmlichen Detergenzien als zusätzlichen Additiven kombinieren.

Als Detergens-Komponente in der Mischung mit den erfindungsgemäßen Stoffen als Dispergatoren kann prinzipiell jedes bekannte der hierfür geeigneten Produkte eingesetzt werden, wie sie z.B. bei J. Falbe, U. Hasserodt, Katalysatoren, Tenside und Mineralöladditive, G. Thieme Verlag Stuttgart 1978, S. 221 f. oder bei K. Owen, Gasoline and Diesel Fuel Additives, John Wiley & Sons 1989, S. 23 ff., beschrieben sind.

Vorzugsweise verwendet man N-haltige Detergenzien, z.B. Verbindungen, die eine Amin- oder Amid-Gruppe enthalten. Insbesondere geeignet sind Polyisobutylamine gemäß EP 0 244 616, Ethylendiamintetraessigsäureamide und/oder -imide gemäß EP 0 188 786 oder Polyetheramine gemäß EP 0 356 725, wobei auf die Definitionen in diesen Literaturstellen Bezug genommen wird. Die dort beschriebenen Produkte verfügen herstellungsbedingt ebenfalls über den Vorteil, chlor- bzw. chloridfrei zu sein.

Soll in erster Linie die Detergens-Wirkung der erfindungsgemäßen β-Aminonitrile und/oder N-Alkyl-1,3-propylendiamine genutzt werden, so können diese Stoffe auch mit Trägerölen kombiniert werden. Derartige Trägeröle sind bekannt, insbesondere eignen sich Trägeröle auf Polyglykolbasis, z.B. entsprechende Ether und/oder Ester, wie sie in der US 5 004 478 oder der DE 38 38 918 A1 beschrieben sind. Auch Polyoxyalkylenmonoole mit Kohlenwasserstoffendgruppen (US 4 877 416) oder Trägeröle, wie sie in der DE 41 42 241.4 offenbart sind, können eingesetzt werden.

Als Kraftstoffe für Ottomotoren kommen verbleites und insbesondere unverbleites Normal- und Superbenzin in Betracht. Die Benzine können auch andere Komponenten als Kohlenwasserstoffe, z.B. Alkohole für Methanol, Ethanol, tert.-Butanol sowie Ether, z.B. Methyltertiärbutylether enthalten. Neben den erfindungsgemäß zu verwendenden alkoxylierten Polyetheraminen enthalten die Kraftstoffe in der Regel noch weitere Zusätze wie Korrosionsinhibitoren, Stabilisatoren, Antioxidantien und/oder weitere Detergentien.

Korrosionsinhibitoren sind meist Ammoniumsalze org. Carbonsäuren, die durch entsprechende Struktur der Ausgangsverbindungen zur Filmbildung neigen. Auch Amine zur Absenkung des pH-Wertes finden sich häufig in Korrosionsinhibitoren. Als Buntmetallkorrosionsschutz werden meist heterocyclische Aromaten eingesetzt.

Die Prüfung der Produkte auf Eignung als Kraftstoffadditive erfolgt mittels Motorentests:

Nach CEC-F-02-T-79 wird die Wirkung als Ventilreiniger getestet.

Ihre Eignung als Dispergatoren für Schmutzpartikel im Motorenöl wurde gemäß DKA-Vorschlag in einem Daimler Benz M 102 E-Motor durchgeführt.

### Beispiele

### 1. Herstellung von N-Polyisobutyl-β-aminopropionitril

Zu 500 g eines Polyisobutylamins (ca. 0,5 mol), hergestellt nach den Angaben von DE-A1 36 11 230 aus einem Polyisobuten einer mittleren Molmasse von 1 000, gelöst in 500 ml eines C₁₂-Kohlenwasserstoff-Gemisches, wurden während ca. 30 min. 24 g (0,45 mol) Acrylnitril zugetropft, wobei die Temperatur bei 70°C gehalten wurde. Danach wurde bei 80°C drei Stunden lang nachgerührt. Die Ausbeute ist nahezu guantitativ. Das so erhaltene Produkt kann für die katalytische Hydrierung direkt eingesetzt werden.

Das Produkt wurde durch folgende Daten charakterisiert:

| Elementaranalyse (Berechnungsgrundlage: MG des Ausgangsprodukts Polyisobutenamin: 1041): | | | |
|---|---|---|---|
| Berechnet: | C: 83,6 % | H: 13,8 % | N: 2,6 % |
| Gefunden: | C: 83,0 % | H: 14,4 % | N: 2,2 % |

- IR-Spektrum:: Bande bei 2230 cm⁻¹ (Nitrilbande)
- ¹H-NMR:: 2 Tripletts bei 2,5 und 2,9 ppm, zuzuordnen den beiden zur Nitrilgruppe α- und β-ständigen CH₂-Gruppen
- ¹³C-NMR:: Signal bei 118,4 ppm (C-Atom der Nitrilgruppe)
Daraus ergibt sich eindeutig, daß es sich bei dem isolierten Produkt um das N-Polyisobutyl-β-aminopropionitril handelt.

### 2. Herstellung von N-Polyisobutyl-1,3-propylendiamin

Die Reaktionsmischung aus der Cyanoethylierung (1.) wurde in einen Autoklaven überführt und mit 5 g Raney-Cobalt sowie 20 g Ammoniak versetzt.

Bei 100°C und 100 bar Druck wurde 20 h lang hydriert. Der Katalysator wurde abgetrennt, danach wurden im Wasserstrahlvakuum leichtflüchtige Bestandteile abgetrennt. Man erhält 550 g eines nahezu farblosen, viskosen Öls mit einer Aminzahl von 93 (Theorie: 90-110).

### 3. Motortestergebnisse nach CEC-F-02-T-79

Prüfungen als Einlaßsystem- und Ventilreiniger

| Produkt | Ablagerungen [mg] Ventil Nr. | | | |
|---|---|---|---|---|
| | 1 | 2 | 3 | 4 |
| Grundwert ohne Additivierung | 530 | 319 | 259 | 651 |
| N-Polyisobutyl-β-aminopropionitril 400 ppm, nach Beispiel 1 | 41 | 29 | 60 | 5 |
| N-Polyisobutyl-1,3-propylendiamin, 400 ppm, nach Beispiel 2 | 2 | 0 | 3 | 0 |

### 4. Prüfung der dispergierenden Wirkung im Daimler Benz M 102 E

Als Prüfkraftstoff wurde ein unverbleiter Superkraftstoff (ROZ 95) nach DIN 51 607 eingesetzt. Das verwendete Motorenöl war das Referenzöl RL 136.

Es wurden Prüfläufe mit nicht additiviertem Benzin (Grundwert) sowie mit 250 ppm an Prüfsubstanz, und als Vergleich hierzu mit 250 ppm Polyisobutylamin gefahren.

Nach Beendigung der Prüfläufe wurde der Motor zerlegt und nach 24 Stunden Abtropfzeit bewertet. Die Bewertung (Rating, Höchstwert 10) der einzelnen Motorenteile für das als Additiv eingesetzte Stoffe aus 1. und 2. enthalten die folgenden Tabellen:

**Tabelle 1**

| Test mit N-Polyisobutyl-β-aminopropionitril | | |
|---|---|---|
| Rating | | |
| Motorenteil | Grundwert | 250 mg/kg N-Polyisobutyl-β-aminopropionitril |
| Zylinderkopfhaube | 8,7 | 8,4 |
| Ölverteilungsrohr | 8,3 | 9,0 |
| Zylinderkopf | 8,3 | 8,9 |
| Ölwanne | 9,2 | 9,4 |
| Steuergehäusedeckel | 8,5 | 9,2 |
| Mittelwert | 8,6 | 9,0 |

Durch die dispergierende Wirkung des Aminonitrils gelingt es, den Mittelwert beim Schlammtest im Mercedes-Benz M 102 E von 8,6 auf 9,0 anzuheben. Die Verwendung von Polyisobutylamin hingegen führte zu keiner Verbesserung gegenüber dem Grundwert.

**Tabelle 2**

| Test mit N-Polyisobutyl-1,3-propylendiamin | | | |
|---|---|---|---|
| Rating | | | Rating |
| Motorenteil | Grundwert | Zusatz von 250 ppm Diamin aus Beispiel 2 | Zusatz von 250 ppm Polyisobutylamin |
| Zylinderkopfhaube | 8,4 | 8,8 | 8,4 |
| Ölverteilungsrohr | 7,6 | 9,5 | 8,0 |
| Zylinderkopf | 7,8 | 9,2 | 8,3 |
| Ölwanne | 8,9 | 9,5 | 8,9 |
| Steuergehäusedeckel | 8,3 | 9,3 | 8,6 |
| Mittelwert | 8,2 | 9,3 | 8,4 |

Die Tabelle zeigt die bessere Wirkung des erfindungsgemäßen Polyisobutyl-propylendiamins im Vergleich zum Polyisobutylamin. Durch die dispergierenden Eigenschaften des Diamins gelingt es, den Mittelwert aus dem Rating der einzelnen Motorenteile von 8,2 auf 9,3 anzuheben. Die Verwendung von Polyisobutylamin hingegen führte nur zu einer geringfügigen Veränderung auf 8.4 Punkte gegenüber dem Grundwert von 8.2.

Die Ergebnisse zeigen, daß die erfindungsgemäßen Stoffe nicht nur eine ausgezeichnete Wirkung als Einlaßsystem- und Ventilreiniger aufweisen, sondern darüber hinaus auch eine sehr gute Schlamm-dispergierende Wirkung zeigen und daher als Additive für Kraftstoffe in besonderem Maße geeignet sind.

## Patentansprüche

1. β-Aminonitrile der folgenden Formel I 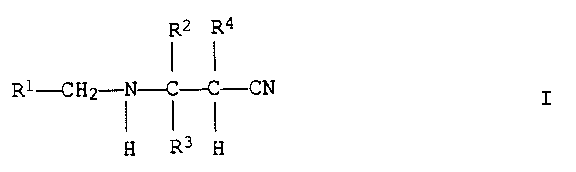 in der
R¹ einen aliphatischen, Alkyl-Seitengruppen aufweisenden Kohlenwasserstoffrest mit einem Molekulargewicht (Zahlenmittel) von 250 bis 5 000 darstellt und
R², R³ und R⁴ unabhängig voneinander Wasserstoff oder einen C₁-C₈-Alkylrest oder R² oder R⁴ einen Phenylrest bedeuten.

2. β-Aminonitrile nach Anspruch 1, dadurch gekennzeichnet, daß R¹ einen von Isobuten und 0 bis 30 Gew.-% n-Buten abgeleitetem Polybutyl- oder Polyisobutylrest bedeutet.

3. β-Aminonitrile nach Anspruch 1, dadurch gekennzeichnet, daß R² und R³ Wasserstoff und R⁴ Wasserstoff oder Methyl bedeuten.

4. N-Alkyl-1,3-propylendiamine der folgenden Formel II 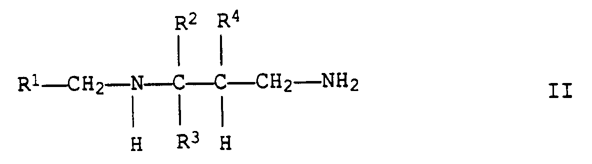 in der R¹ bis R⁴ die in Anspruch 1 angegebene Bedeutung haben, dadurch gekennzeichnet, daß sie durch Hydrierung der β-Aminonitrile der Formel I nach Anspruch 1 an üblichen Hydrierkatalysatoren unter Druck bei Temperaturen von 50°C bis 300°C in Gegenwart von NH₃ erhalten werden.

5. N-Alkyl-1,3-propylendiamine der Formel II nach Anspruch 4, dadurch gekennzeichnet, daß sie durch Hydrierung von β-Aminonitrilen nach Anspruch 2 erhalten werden.

6. Verfahren zur Herstellung von β-Aminonitrilen der Formel I nach Anspruch 1, dadurch gekennzeichnet, daß man Amine der Formel III
R¹―CH₂―NH₂ III
mit Nitrilen der allgemeinen Formel IV 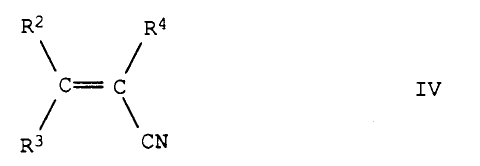 gegebenenfalls in Gegenwart eines Katalysators umsetzt, wobei R¹ bis R⁴ die in Anspruch 1 angegebenen Bedeutungen haben.

7. Verfahren zur Herstellung der N-Alkyl-1,3-propylendiamine nach Anspruch 4, dadurch gekennzeichnet, daß man β-Aminonitrile der Formel I an üblichen Hydrierkatalysatoren unter Druck bei Temperaturen von 50°C bis 300°C in Gegenwart von NH₃ hydriert.

8. Kraftstoffe für Ottomotoren, dadurch gekennzeichnet, daß sie ein β-Aminonitril der Formel I gemäß Anspruch 1 und/oder ein N-Alkyl-1,3-propylendiamin der Formel II gemäß Anspruch 4 in wirksamen Mengen enthalten.

9. Schmierstoffe, dadurch gekennzeichnet, daß sie ein β-Aminonitril der Formel I gemäß Anspruch 1 und/oder ein N-Alkyl-1,3-propylendiamin der Formel II gemäß Anspruch 4 in wirksamen Mengen enthalten.

10. Verwendung der β-Aminonitrile gemäß Anspruch 1 und/oder der N-Alkyl-1,3-propylendiamine gemäß Anspruch 4 als Additive für Kraft- oder Schmierstoffe.

## Claims

1. A β-aminonitrile of the following formula I 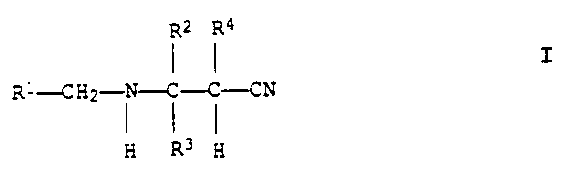 where R¹ is an aliphatic hydrocarbon radical having alkyl side groups and a number average molecular weight of from 250 to 5,000 and R², R³ and R⁴, independently of one another, are each hydrogen or C₁-C₈-alkyl or R² or R⁴ is phenyl.

2. A β-aminonitrile as claimed in claim 1, wherein R¹ is a polybutyl or polyisobutyl radical derived from isobutene and 0-30% by weight of n-butene.

3. A β-aminonitrile as claimed in claim 1, wherein R² and R³ are each hydrogen and R⁴ is hydrogen or methyl.

4. An N-alkyl-1,3-propylenediamine of the following formula II 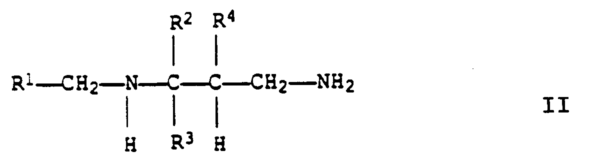 where R¹ to R⁴ have the meanings stated in claim 1, which is obtained by hydrogenating a β-aminonitrile of the formula I as claimed in claim 1 over a conventional hydrogenation catalyst under superatmospheric pressure at from 50 to 300°C in the presence of NH₃.

5. An N-alkyl-1,3-propylenediamine of the formula II as claimed in claim 4, which is obtained by hydrogenating a β-aminonitrile as claimed in claim 2.

6. A process for the preparation of a β-aminonitrile of the formula I as claimed in claim 1, wherein an amine of the formula III
R¹-CH₂-NH₂ III
is reacted with a nitrile of the formula IV 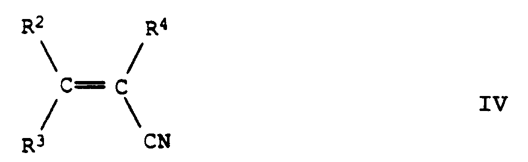 in the presence or absence of a catalyst, R¹ to R⁴ having the meanings stated in claim 1.

7. A process for the preparation of an N-alkyl-1,3-propylenediamine as claimed in claim 4, wherein a β-aminonitrile of the formula I is hydrogenated over a conventional hydrogenation catalyst under super-atmospheric pressure at from 50 to 300°C in the presence of NH₃.

8. A fuel for gasoline engines, which contains a β-aminonitrile of the formula I as claimed in claim 1 and/or an N-alkyl-1,3-propylenediamine of the formula II as claimed in claim 4 in effective amounts.

9. A lubricant which contains a β-aminonitrile of the formula I as claimed in claim 1 and/or an N-alkyl-1,3-propylenediamine of the formula II as claimed in claim 4 in effective amounts.

10. Use of a β-aminonitrile as claimed in claim 1 and/or of an N-alkyl-1,3-propylenediamine as claimed in claim 4 as additives for fuels or lubricants.

## Revendications

1. β-Aminonitriles de formule I 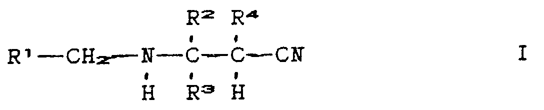 dans laquelle
R¹ représente un reste hydrocarboné aliphatique comportant des groupements latéraux alkyle et ayant un poids moléculaire (moyenne en nombre) de 250 à 5000, et
R², R³ et R⁴ représentent chacun, indépendamment les uns des autres, un atome d'hydrogène ou un reste alkyle en C₁-C₈, ou bien R² ou R⁴ représente un reste phényle.

2. β-Aminonitriles selon la revendication 1, caractérisés en ce que R¹ représente un reste polybutyle ou polyisobutyle dérivé d'isobutylène et de 0 à 30% en poids de n-butylène.

3. β-Aminonitriles selon la revendication 1, caractérisés en ce que R² et R³ représentent des atomes d'hydrogène et R⁴ représente un atome d'hydrogène ou un reste méthyle.

4. N-Alkyl-1,3-propylènediamines de formule II 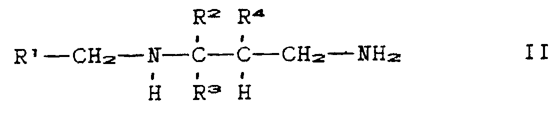 dans laquelle R¹ à R⁴ ont les mêmes significations que dans la revendication 1, caractérisées en ce qu'elles sont obtenues par hydrogénation des β-aminonitriles de formule I selon la revendication 1 en présence de catalyseurs d'hydrogénation usuels, sous pression, à des températures de 50 à 300°C et en présence de NH₃.

5. N-Alkyl-1,3-propylènediamines de formule II selon la revendication 4, caractérisées en ce qu'elles sont obtenues par hydrogénation de β-aminonitriles selon la revendication 2.

6. Procédé de préparation de β-aminonitriles de formule I selon la revendication 1, caractérisé en ce que l'on fait réagir des amines de formule III
R¹―CH₂―NH₂ III
avec des nitriles de formule générale IV 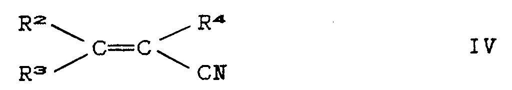 éventuellement en présence d'un catalyseur, R¹ à R⁴ ayant les mêmes significations que dans la revendication 1.

7. Procédé de préparation des N-alkyl-1,3-propylènediamines selon la revendication 4, caractérisé en ce que l'on hydrogène des β-aminonitriles de formule I en présence de catalyseurs d'hydrogénation usuels, sous pression, à des températures de 50 à 300°C et en présence de NH₃.

8. Carburants pour moteurs à allumage par étincelle, caractérisés en ce qu'ils contiennent, dans des quantités efficaces, un β-aminonitrile de formule I selon la revendication 1 et/ou une N-alkyl-1,3-propylènediamine de formule II selon la revendication 4.

9. Lubrifiants, caractérisés en ce qu'ils contiennent, dans des quantités efficaces, un β-aminonitrile de formule I selon la revendication 1 et/ou une N-alkyl-1,3-propylènediamine de formule II selon la revendication 4.

10. Uilisation des β-aminonitriles selon la revendication 1 et/ou des N-alkyl-1,3-propylènediamines selon la revendication 4 comme additifs pour des carburants ou des lubrifiants.
